(19) Europäisches Patentamt
European Patent Office
Office européen des brevets

(11) Veröffentlichungsnummer: **0 059 687**
**B1**

(12) # EUROPÄISCHE PATENTSCHRIFT

(45) Veröffentlichungstag der Patentschrift: **10.04.85**

(51) Int. Cl.⁴: **C 07 C 43/295,** C 07 C 121/75, C 07 C 47/575

(21) Anmeldenummer: **82810086.7**

(22) Anmeldetag: **22.02.82**

(54) **3-Phenoxybenzylverbindungen, Verfahren zu ihrer Herstellung und ihre Verwendung zur Synthese von bioziden Verbindungen.**

(30) Priorität: **26.02.81 CH 1296/81**
**09.11.81 CH 7169/81**

(43) Veröffentlichungstag der Anmeldung: **08.09.82 Patentblatt 82/36**

(45) Bekanntmachung des Hinweises auf die Patenterteilung: **10.04.85 Patentblatt 85/15**

(84) Benannte Vertragsstaaten: **AT BE CH DE FR GB IT LI NL**

(56) Entgegenhaltungen:
**EP - A - 0 008 333**
**EP - A - 0 009 708**

(73) Patentinhaber: **CIBA-GEIGY AG, Postfach, CH-4002 Basel (CH)**

(72) Erfinder: **Ackermann, Peter, Dr., Reichensteinerstrasse 12, CH-4153 Reinach (CH)**
Erfinder: **Gsell, Laurenz, Dr., Maiengasse 56, CH-4056 Basel (CH)**
Erfinder: **Wehrli, Rudolf, Dr., Dianastrasse 2, CH-4310 Rheinfelden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 059 687 B1**

ACTORUM AG

**Beschreibung**

Die vorliegende Erfindung betrifft 3-Phenoxybenzylverbindungen, Verfahren zu ihrer Herstellung und ihre Verwendung als Zwischenproukte zur Synthese von bioziden Verbindungen.

Die 3-Phenoxybenzylverbindungen haben die Formel

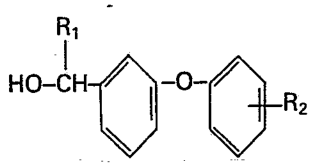

(I),

worin $R_1$ Wasserstoff, Cyano, $-CSNH_2$, Allenyl, $C_2$-$C_3$-Alkenyl oder $C_2$-$C_3$-Alkinyl und $R_2$ gegebenenfalls durch Cyano, Halogen, $C_1$-$C_6$-Alkyl, $C_1$-$C_6$-Hydroxyalkyl, $C_1$-$C_6$-Halogenalkyl oder Dimethylamino, substituiertes Alkenyl oder Alkinyl bedeuten.

Die für $R_1$ und $R_2$ in Frage kommenden Alkenyl- oder Alkinylgruppen können geradkettig oder verzweigt sein, wobei die 2fach- respektive 3fach-Bindung bei $R_2$ mit dem Phenylkern konjugiert ist. Die Alkenyl- oder Alkinylgruppen bei $R_2$ haben in der Kette bevorzugt 2 bis 6, insbesondere aber 2 bis 3, Kohlenstoffatome. Unter Halogen ist Fluor, Chlor, Brom oder Jod, insbesondere Brom oder Jod, zu verstehen.

Als Zwischenprodukte interessant sind Verbindungen der Formel I, worin $R_1$ Wasserstoff, Cyano, Allenyl oder Äthinyl und $R_2$ p-Vinyl, p-1-Propenyl, p-3-Dimethylamino-1-propenyl, p-Äthinyl, p-1-Propinyl, p-Jodäthinyl oder p-3-Dimthylamino-1-propinyl bedeuten.

Die Verbindungen der Formel I können nach an sich bekannten Methoden z.B. wie folgt hergestellt werden:

Besonders interessant sind aber Verbindungen der Formel I, worin $R_1$ Wasserstoff oder Cyano und $R_2$ p-Vinyl, p-1-Propenyl, p-Äthinyl oder p-1-Propinyl bedeuten.

In der europäischen Patentanmeldung Nr. 0008333 werden α-Prop-1-inyl-3-phenoxybenzylalkohole, welche am Phenoxyteil durch Halogen, $C_1$-$C_4$-Alkyl, $C_1$-$C_3$-Alkoxy, Cyano oder Dimethylamino substituiert sein können und in der europäischen Patentanmeldung Nr. 0009708 Phenoxybenzylalkohole beschrieben, welche wenigstens an einer der beiden Phenylgruppen durch Halogen substituiert sind. Sowohl die α-Prop-1-inyl-benzylalkohole der europäischen Patentanmeldung Nr. 0008333 als auch die Phenoxybenzylalkohole der europäischen Patentanmeldung Nr. 0009708 werden als Zwischenprodukte zur Herstellung von pyrethrinartigen Verbindungen verwendet.

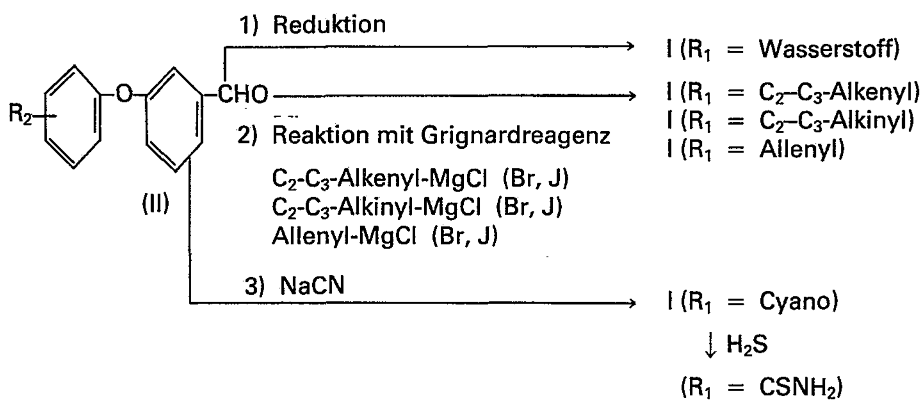


In der Formel II hat $R_2$ die für Formel I angegebene Bedeutung.

Die Verfahren werden bei einer Reaktionstemperatur zwischen −10 bis 100°C, vorzugsweise zwischen 0 bis 80°C, im allgemeinen bei Normaldruck und gegebenenfalls in einem inerten Lösungs- oder Verdünnungsmittel durchgeführt. Als Lösungs- oder Verdünnungsmittel eignen sich vor allem Äther wie Diäthyläther, Tetrahydrofuran oder Dioxan für das zweite Verfahren, während für die Verfahren eins und drei vorzugsweise Alkohole wie Methanol oder Äthanol verwendet werden.

Die Verbindungen der Formel II, welche nach bekannten Methoden hergestellt werden können, sind neu und bilden ebenfalls einen Gegenstand der vorliegenden Erfindung.

Die Verbindungen der Formel I eignen sich als Zwischenprodukte für die Herstellung von Verbindungen wie z.B. von Pyrethroiden (vgl. Bsp. 2), welche sich zur Bekämpfung von Vertretern des Stammes Arthropoda, wie der Klassen Insekta und Arachnoida, eignen.

Beispiel 1:
Herstellung der m-(p-Äthinylphenoxy)-benzylalkohole Reaktionsschema

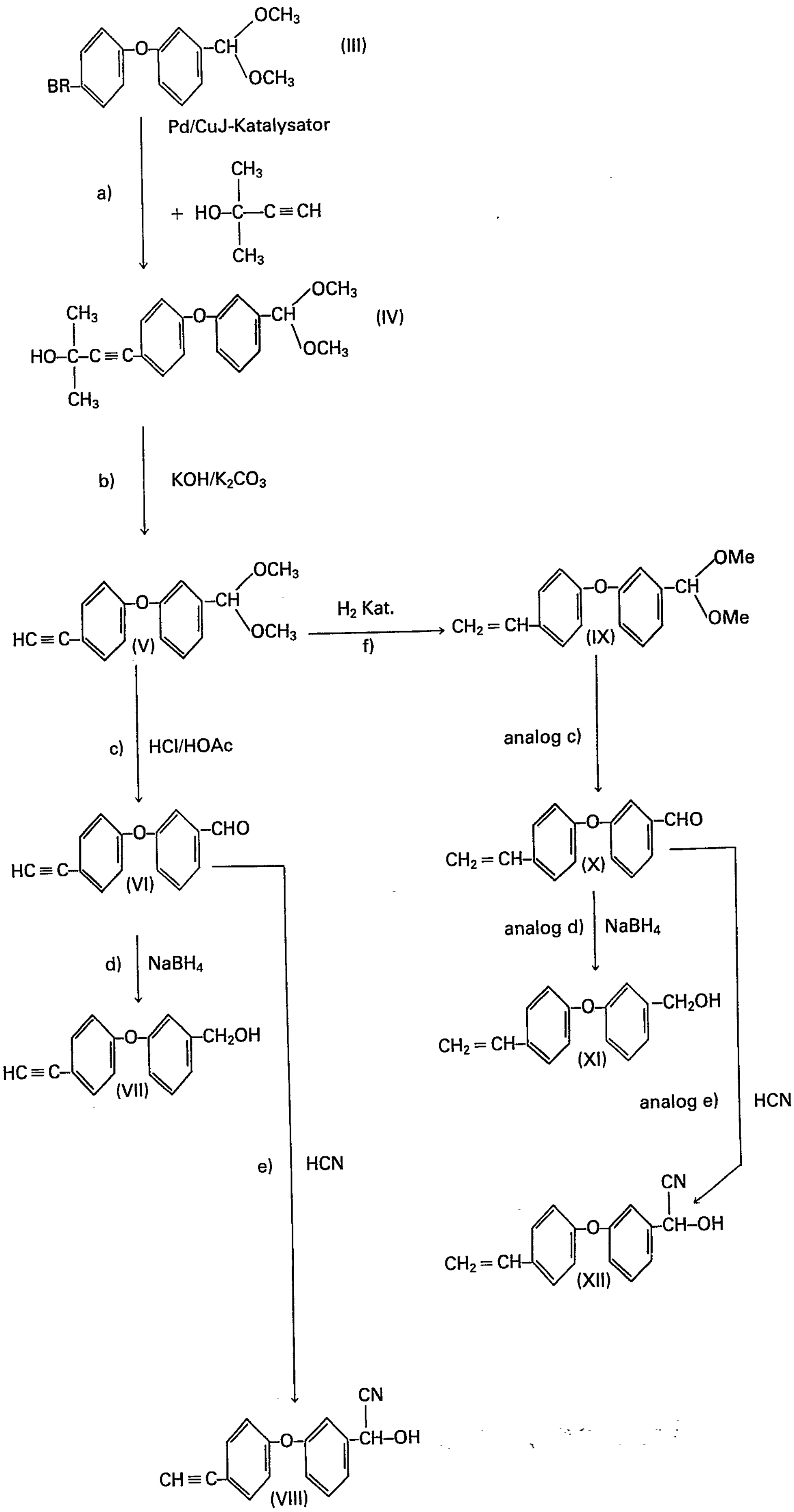
(III)
Pd/CuJ-Katalysator
a)
(IV)
b)
KOH/$K_2CO_3$
(V)
$H_2$ Kat.
f)
(IX)
c)
HCl/HOAc
analog c)
(VI)
(X)
d)
$NaBH_4$
analog d)
$NaBH_4$
(VII)
(XI)
analog e)
HCN
e)
HCN
(XII)
(VIII)

a) 33,5 g der Verbindung der Formel III, 13,1 g 2-Methyl-3-butin-2-ol, 104 ml Triäthylamin, 0,73 g $(Ph_3P)\ PdCl_2$ und 0,4 CuJ werden zusammen gegeben und auf 80°C aufgeheizt. Nach 16stündigem Rühren bei 80°C wird filtriert und mit Äther extrahiert. Die Ätherphase wird mit Ammoniumchlorid- und anschliessend mit Kochsalzlösung gewaschen, über Magnesiumsulfat getrocknet und eingeengt.

Nach der Chromatographie des Rohproduktes über Kieselgel mit Äther/Hexan (1:1) als Eluiermittel erhält man die Verbindung der Formel IV mit einer Refraktion von $n_D^{20°} = 1{,}5445$.

b) 50 g der Verbindung der Formel IV, 5 g KOH, 50 g $K_2CO_3$ in 500 ml Toluol werden während einer Stunde auf 140°C aufgeheizt. Nach dem Abdestillieren des Toluols und der Chromatographie über Kieselgel mit Hexan/Äther (10:1) als Eluiermittel erhält man die Verbindung der Formel V mit einer Refraktion von $n_D^{20°} = 1{,}5748$.

c) 17,9 g der Verbindung der Formel V, 67 ml 2n HCl und 33,5 ml Eisessig werden 2 Stunden bei 60°C gerührt. Das Reaktionsgemisch wird auf Natriumacetat gegossen und mit Essigester extrahiert.

Die Essigesterphase wird zweimal mit Natriumacetat und einmal mit gesättigter Kochsalzlösung gewaschen, über Magnesiumsulfat getrocknet und eingeengt.

Das Rohprodukt wird über Kieselgel mit Hexan/Äther (5:1) als Eluiermittel chromatographiert. Man erhält die Verbindung der Formel VI mit einer Refraktion von $n_D^{20°} = 1{,}6009$.

d) 0,18 g $NaBH_4$ und 1,4 ml Wasser werden unter Stickstoff vorgelegt. Unter Kühlung werden 3,5 g des Aldehyds der Formel VI in 11,4 ml Methanol langsam zugetropft. Nach 12stündigem Rühren bei 20°C wird mit Wasser verdünnt und zweimal mit $CH_2Cl_2$ extrahiert. Die organische Phase wird mit gesättigter Kochsalzlösung gewaschen und über Magnesiumsulfat getrocknet.

Das Rohprodukt wird über Kieselgel mit Hexan/Äther (1:1) als Eluiermittel chromatographiert. Man erhält die Verbindung der Formel VII mit einer Refraktion von $n_D^{20°} = 1{,}5942$.

e) Zu 3,3 g Natriumcyanid in 4 ml Wasser und 87 ml Methanol werden bei 0°C zuerst 10 g des Aldehydes der Formel (VI)

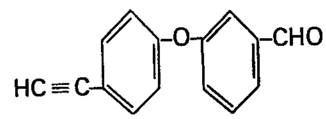

in 10 ml Methanol und anschliessend 10,3 ml Essigsäure zugetropft. Nach zweistündigem Rühren bei 20°C wird das Reaktionsgemisch auf Eiswasser gegossen und mit $CH_2Cl_2$ extrahiert. Die organische Phase einmal mit Natriumacetat extrahiert und zweimal mit Eiswasser gewaschen, über Magnesiumsulfat getrocknet, filtriert und eingeengt.

Man erhält den gewünschten α-Cyanobenzylalkohol (Formel VIII) mit einer Refraktion von $n_D^{20°} = 1{,}6049$.

f) In eine Lösung von 10 g der Verbindung der Formel V in 100 ml Dimethylformamid mit 1 g Lindlar-Katalysator wird bei 20°C Wasserstoff eingeleitet. Nachdem 1 Äquivalent Wasserstoff aufgenommen ist, wird das Reaktionsgemisch filtriert und das Lösungsmittel unter vermindertem Druck entfernt.

Man erhält die Verbindung der Formel IX mit einer Refraktion von $n_D^{20°} = 1{,}5828$.

Analog dem Verfahrensschritt c) erhält man aus der Verbindung der Formel IX die Verbindung der Formel X mit einer Refraktion von $n_D^{30°} = 1{,}6128$. Aus dem Aldehyd der Formel X erhält man analog dem Verfahrensschritt d) die Verbindung der Formel XI mit einer Refraktion von $n_D^{30°} = 1{,}6061$ sowie analog dem Verfahrensschritt e) aus der Verbindung XI die Verbindung der Formel XII mit einer Refraktion von $n_D^{30°} = 1{,}5963$.

Auf analoge Weise und nach bekannten Methoden werden auch folgende Verbindungen hergestellt:

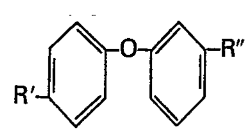

| R′ | R″ | physikalische Daten |
|---|---|---|
| $CH_3$–C≡C– | $OCH_3$<br>/<br>–CH<br>\\<br>$OCH_3$ | $n_D^{20°} = 1{,}5911$ |
| $CH_3$–C≡C– | –CHO | $n_D^{30°} = 1{,}6212$ |
| $CH_3$–C≡C– | CN<br>\|<br>–CH–OH | $n_D^{20°} = 1{,}6071$ |
| $CH_3$–C≡C– | C≡CH<br>\|<br>–CH–OH | $n_D^{20°} = 1{,}6159$ |

| R' | R'' | physikalische Daten |
|---|---|---|
| $CH_3-C\equiv C-$ | 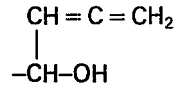 | $n_D^{20°} = 1,6128$ |
| $CH_3-CH=CH-$ | $-CH_2OH$ | $n_D^{20°} = 1,6057$ |
| $CH_3-CH=CH-$ | 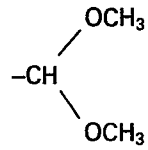 | $n_D^{20°} = 1,5790$ |
| $CH_3-CH=CH-$ | $-CHO$ | $n_D^{20°} = 1,640$ |
| $CH_3-CH=CH-$ | 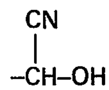 | $n_D^{20°} = 1,6094$ |
| $J-C\equiv C-$ | 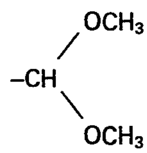 | Smp.: 72 bis 74°C |
| $J-C\equiv C-$ | $-CHO$ | Smp.: 106 bis 111°C |
| $J-C\equiv C-$ | 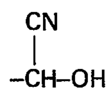 | $n_D^{20°} = 1,6013$ |
| $(CH_3)_2N-CH_2-CH=CH-$ | $-CHO$ | $n_D^{20°} = 1,5922$ |
| $(CH_3)_2N-CH_2-CH=CH-$ | 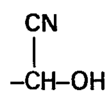 | $n_D^{20°} = 1,5687$ |
| $(CH_3)_2N-CH_2-C\equiv C-$ | $-CHO$ | $n_D^{20°} = 1,6018$ |
| $(CH_3)_2N-CH_2-C\equiv C-$ | 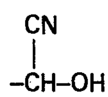 | $n_D^{20°} = 1,6220$ |

Beispiel 2:
Herstellung der bioziden Verbindung der Formel

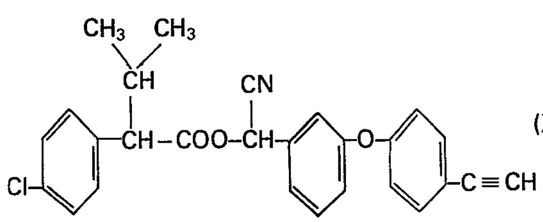

(XIII)

Zu einer Lösung von 2,3 g der Verbindung der Formel

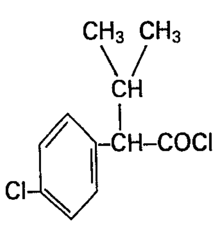

in 20 ml Toluol werden bei 0°C zuerst 1 ml Pyridin und anschliessend 5,4 g der Verbindung der Formel

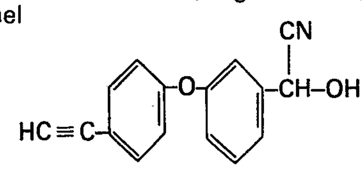

gelöst in 20 ml Toluol zugetropft.

Nach 12stündigem Rühren bei 20°C wird das Reaktionsgemisch auf 2n Salzsäure gegossen, einmal mit 10%iger Kaliumkarbonatlösung, einmal gesättigter Natriumkarbonatlösung und einmal mit gesättigter Kochsalzlösung extrahiert und anschliessend mit Magnesiumsulfat getrocknet. Nach dem Filtrieren und Einengen wird das Rohprodukt über Kieselgel mit Toluol als Eluiermittel chromatographiert.

Man erhält die Verbindung der Formel XIII mit einer Refraktion von $n_D^{20°} = 1{,}5811$.

**Patentansprüche für die Vertragsstaaten: BE, CH, DE, FR, GB, LI, IT, NL**

1. 3-Phenoxybenzylalkohole der Formel

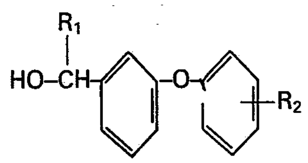

worin $R_1$ Wasserstoff, Cyano, $-CSNH_2$, Allenyl, $C_2$-$C_3$-Alkenyl oder $C_2$-$C_3$-Alkinyl und $R_2$ gegebenenfalls durch Cyano, Halogen, $C_1$-$C_6$-Alkyl, $C_1$-$C_6$-Hydroxyalkyl, $C_1$-$C_6$-Halogenalkyl oder Dimethylamino substituiertes Alkenyl oder Alkinyl bedeuten.

2. Verbindungen gemäss Anspruch 1, worin $R_1$ Wasserstoff, Cyano, Allenyl oder Äthinyl und $R_2$ p-Vinyl, p-1-Propenyl, p-3-Dimethylamino-1-propenyl, p-Äthinyl, p-1-Propinyl, p-Jodäthinyl oder p-3-Dimethylamino-1-propinyl bedeuten.

3. Verbindungen gemäss Anspruch 2, worin $R_1$ Wasserstoff oder Cyano und $R_2$ p-Vinyl, p-1-Propenyl, p-Äthinyl oder p-1-Propinyl bedeuten.

4. Die Verbindung gemäss Anspruch 3 der Formel

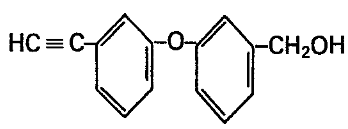

5. Die Verbindung gemäss Anspruch 3 der Formel

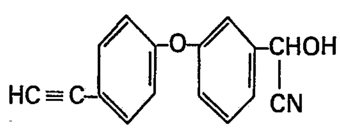

6. Die Verbindung gemäss Anspruch 3 der Formel

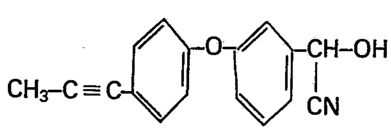

7. Die Verbindung gemäss Anspruch 2 der Formel

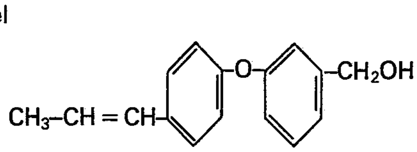

8. Die Verbindung gemäss Anspruch 2 der Formel

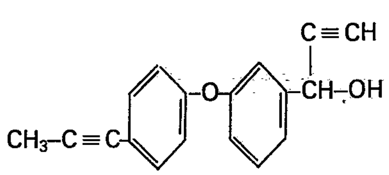

9. Die Verbindung gemäss Anspruch 2 der Formel

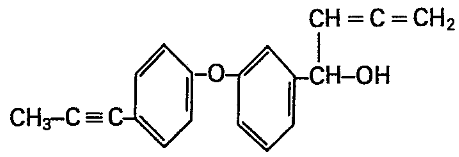

10. Die Verbindung gemäss Anspruch 3 der Formel

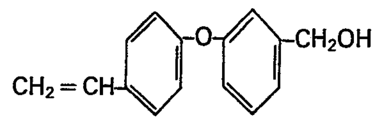

11. Die Verbindung gemäss Anspruch 3 der Formel

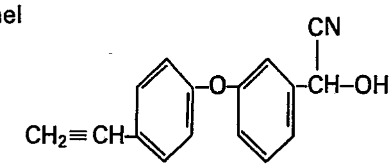

12. Die Verbindung gemäss Anspruch 3 der Formel

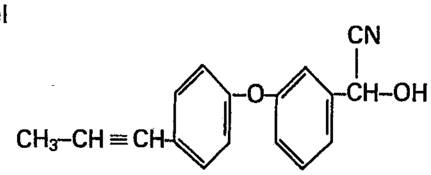

13. Die Verbindung gemäss Anspruch 2 der Formel

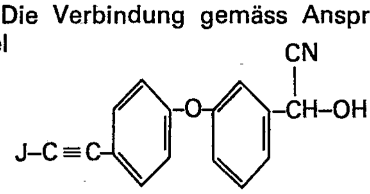

14. Die Verbindung gemäss Anspruch 2 der Formel

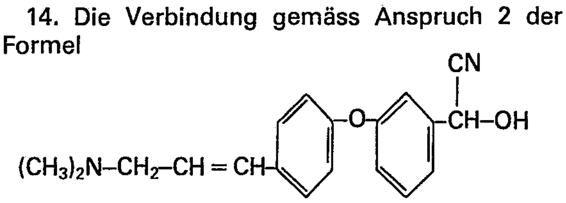

15. Die Verbindung gemäss Anspruch 2 der Formel

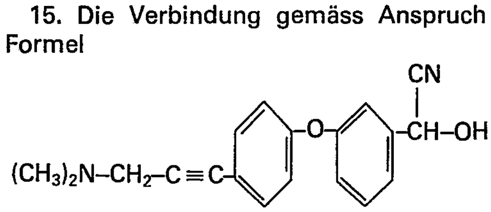

16. Verfahren zur Herstellung einer Verbindung gemäss Anspruch 1, dadurch gekennzeichnet, dass man die Verbindung der Formel

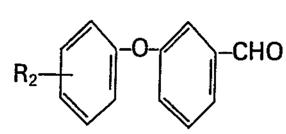

worin $R_2$ die im Anspruch 1 angegebene Bedeutung hat,

a) zu einer Verbindung der Formel

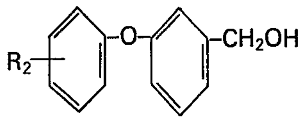

($R_1$ = Wasserstoff)

reduziert oder

b) zuerst mit NaCN zu einer Verbindung der Formel

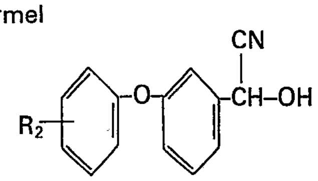

($R_1$ = Cyano)

und diese Verbindung anschliessend mit Schwefelwasserstoff zu einer Verbindung der Formel

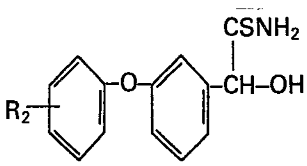

($R_1$ = $-CSNH_2$)

umsetzt, oder

c) mit $C_2$-$C_3$-Alkenyl-MgCl (Br, J), $C_2$-$C_3$-Alkinyl-MgCl (Br, J) oder Allenyl-MgCl (Br, J) zu einer Verbindung der Formel

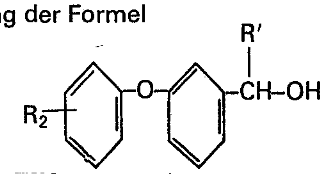

umsetzt, worin $R_2$ die im Anspruch 1 angegebene Bedeutung hat und R′ für $C_2$-$C_3$-Alkenyl, $C_2$-$C_3$-Alkinyl oder Allenyl steht.

17. 3-Phenoxybenzaldehyde der Formel

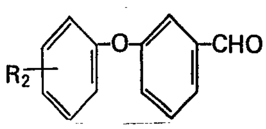

worin $R_2$ die in Anspruch 1 angegebene Bedeutung hat.

**Patentansprüche für den Vertragsstaat: AT**

1. Verfahren zur Herstellung von 3-Phenoxybenzylalkoholen der Formel

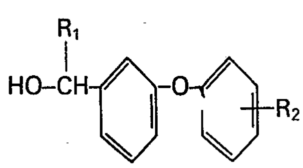

worin $R_1$ Wasserstoff, Cyano, $-CSNH_2$, Allenyl, $C_2$-$C_3$-Alkenyl oder $C_2$-$C_3$-Alkinyl und $R_2$ gegebenenfalls durch Cyano, Halogen, $C_1$-$C_6$-Alkyl, $C_1$-$C_6$-Hydroxyalkyl, $C_1$-$C_6$-Halogenalkyl oder Dimethylamino substituiertes Alkenyl oder Alkinyl bedeuten, dadurch gekennzeichnet, dass man eine Verbindung der Formel

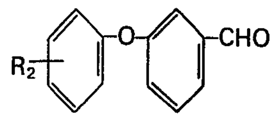

worin $R_2$ die oben angegebene Bedeutung hat,

a) zu einer Verbindung der Formel

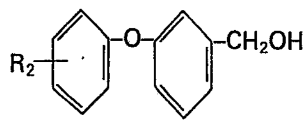

($R_1$ = Wasserstoff)

reduziert oder

b) zuerst mit NaCN zu einer Verbindung der Formel

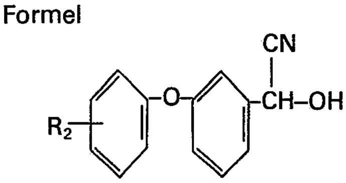

($R_1$ = Cyano)

und diese Verbindung mit Schwefelwasserstoff zu einer Verbindung der Formel

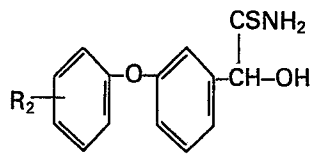

($R_1$ = $-CSNH_2$)

umsetzt, oder

c) mit Alkenyl-MgCl (Br, J), Alkinyl-MgCl (Br, J) oder Allenyl-MgCl (Br, J) zu einer Verbindung der Formel

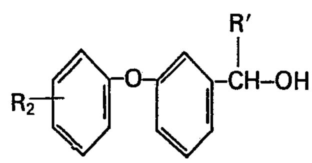

umsetzt, worin $R_1$ für Alkenyl, Alkinyl oder Allenyl steht.

2. Verfahren zur Herstellung gemäss Anspruch 1 von 3-Phenoxy-benzylalkoholen der Formel

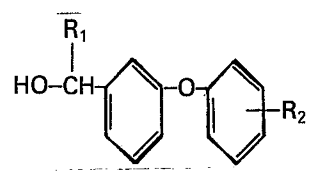

worin $R_1$ Wasserstoff, Cyano, Allenyl oder Äthinyl und $R_2$ p-Vinyl, p-1-Propenyl, p-3-Dimethylamino-1-propenyl, p-Äthinyl, p-1-Propinyl, p-Jodäthinyl oder p-3-Dimethylamino-1-propinyl bedeuten.

3. Verfahren zur Herstellung gemäss Anspruch 2 von 3-Phenoxybenzylalkoholen der Formel

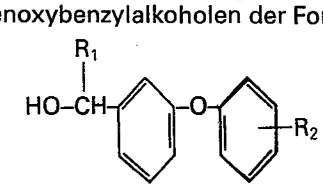

worin $R_1$ Wasserstoff oder Cyano und $R_2$ p-Vinyl, p-1-Propenyl, p-Äthinyl oder p-1-Propinyl bedeuten.

4. Verfahren zur Herstellung gemäss Anspruch 3 der Verbindung der Formel

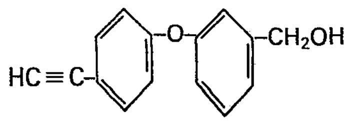

5. Verfahren zur Herstellung gemäss Anspruch 3 der Verbindung der Formel

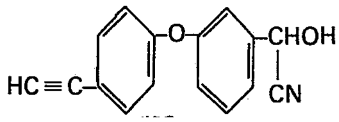

6. Verfahren zur Herstellung gemäss Anspruch 3 der Verbindung der Formel

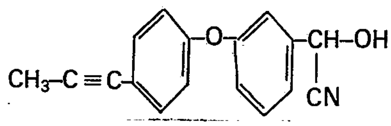

7. Verfahren zur Herstellung gemäss Anspruch 2 der Verbindung der Formel

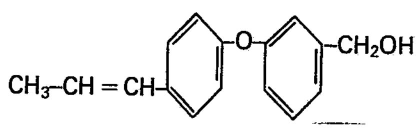

8. Verfahren zur Herstellung gemäss Anspruch 2 der Verbindung der Formel

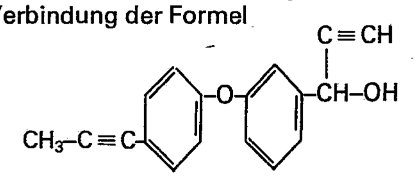

9. Verfahren zur Herstellung gemäss Anspruch 2 der Verbindung der Formel

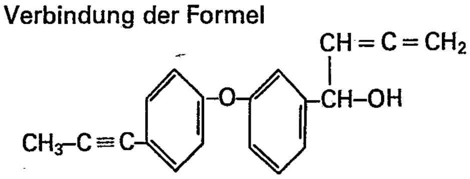

10. Verfahren zur Herstellung gemäss Anspruch 3 der Verbindung der Formel

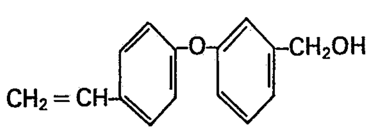

11. Verfahren zur Herstellung gemäss Anspruch 3 der Verbindung der Formel

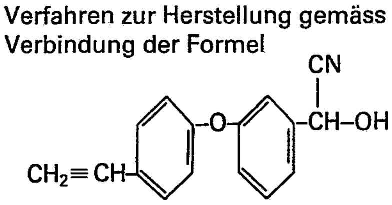

12. Verfahren zur Herstellung gemäss Anspruch 3 der Verbindung der Formel

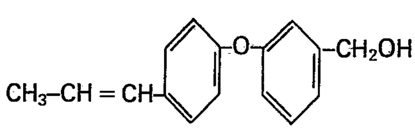

13. Verfahren zur Herstellung gemäss Anspruch 2 der Verbindung der Formel

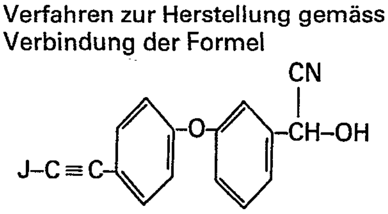

14. Verfahren zur Herstellung gemäss Anspruch 2 der Verbindung der Formel

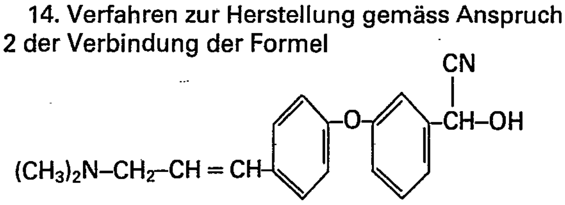

15. Verfahren zur Herstellung gemäss Anspruch 2 der Verbindung der Formel

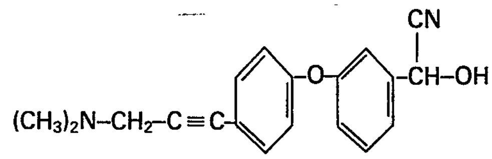

**Claims for the Contracting States: BE, CH, DE, FR, GB, LI, IT, NL**

1. A 3-phenoxybenzyl alcohol of the formula

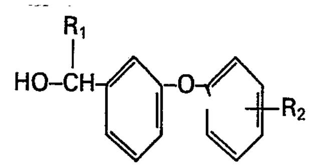

wherein $R_1$ is hydrogen, cyano, $-CSNH_2$, allenyl, $C_2$-$C_3$alkenyl or $C_2$-$C_3$alkynyl, and $R_2$ is alkenyl or alkynyl, each unsubstituted or substituted by cyano, halogen, $C_1$-$C_6$alkyl, $C_1$-$C_6$hydroxyalkyl, $C_1$-$C_6$haloalkyl or dimethylamino.

2. A compound according to claim 1, wherein $R_1$ is hydrogen, cyano, allenyl or ethynyl, and $R_2$ is p-vinyl, p-propen-1-yl, p-3-dimethylaminopropen-1-yl, p-ethynyl, p-propyn-1-yl, p-iodoethynyl or p-3-dimethylaminopropyn-1-yl.

3. A compound according to claim 2, wherein $R_1$ is hydrogen or cyano, $R_2$ is p-vinyl, p-propen-1-yl, p-ethynyl or p-propyn-1-yl.

4. The compound according to claim 3 of the formula

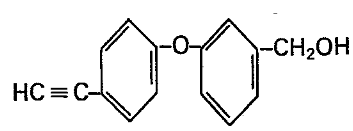

5. The compound according to claim 3 of the formula

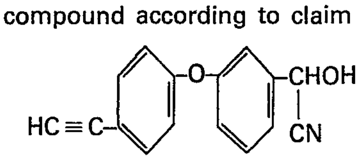

6. The compound according to claim 3 of the formula

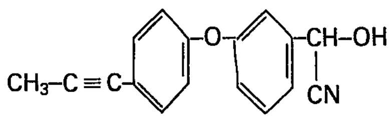

7. The compound according to claim 2 of the formula

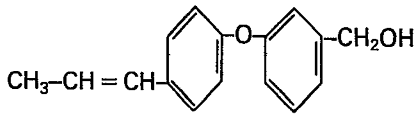

8. The compound according to claim 2 of the formula

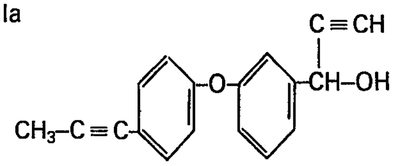

9. The compound according to claim 2 of the formula

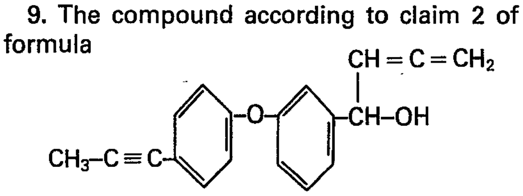

10. The compound according to claim 3 of the formula

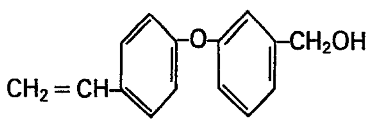

11. The compound according to claim 3 of the formula

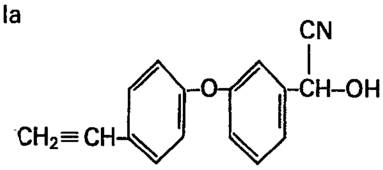

12. The compound according to claim 3 of the formula

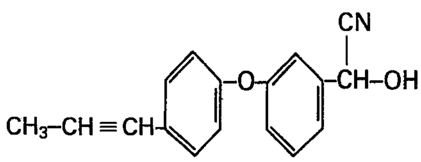

13. The compound according to claim 2 of the formula

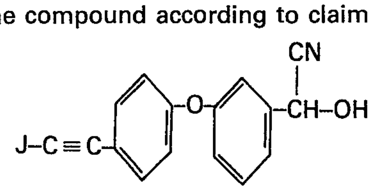

14. The compound according to claim 2 of the formula

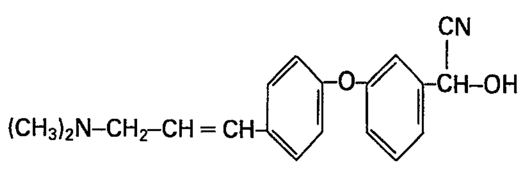

15. The compound according to claim 2 of the formula

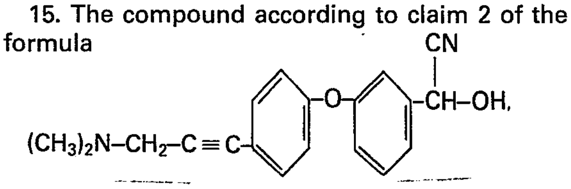

16. A process for the production of a compound according to claim 1, wherein a compound of formula

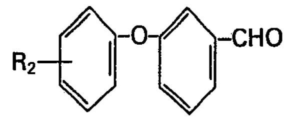

wherein $R_2$ is as defined in claim 1.

a) is reduced to a compound of the formula

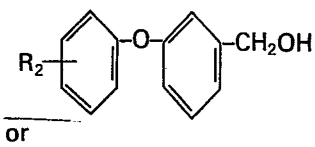 ($R_1$ = hydrogen)

or

b) is reacted first with NaCN to give a compound of the formula

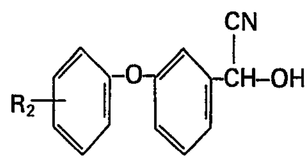 ($R_1$ = cyano)

and subsequently with hydrogen sulfide to give a compound of the formula

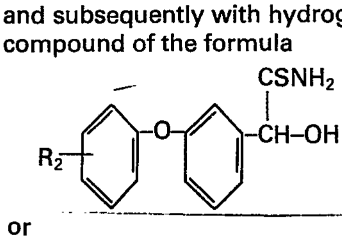 ($R_1$ = $-CSNH_2$)

or

c) is reacted with $C_2$-$C_3$alkenyl-MgCl (Br or I), $C_2$-$C_3$alkynyl-MgCl (Br or I), or allenyl-MgCl (Br or I) to give a compound of the formula

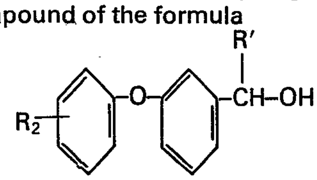

wherein $R_2$ is as defined in claim 1 and R′ is $C_2$-$C_3$alkenyl, $C_2$-$C_3$alkynyl or allenyl.

17. A 3-phenoxybenzaldehyde of the formula

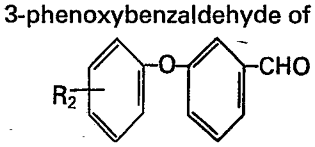

wherein $R_2$ is as defined in claim 1.

**Claims for the Contracting State: AT**

1. A process for the preparation of a 3-phenoxybenzyl alcohol of the formula

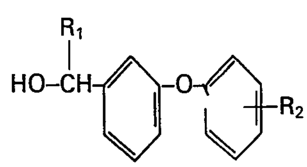

wherein $R_1$ is hydrogen, cyano, $-CSNH_2$, allenyl, $C_2$-$C_3$alkenyl or $C_2$-$C_3$alkynyl, and $R_2$ is alkenyl or alkynyl, each unsubstituted or substituted by cyano, halogen, $C_1$-$C_6$alkyl, $C_1$-$C_6$hydroxyalkyl, $C_1$-$C_6$haloalkyl or dimethylamino, wherein a compound of the formula

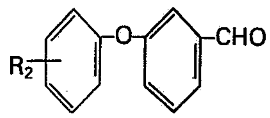

wherein $R_2$ is as defined in claim 1,

a) is reduced to a compound of the formula

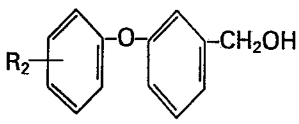

($R_1$ = hydrogen)

or

b) is reacted first with NaCN to give a compound of the formula

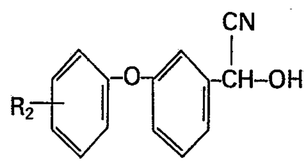

($R_1$ = cyano)

and subsequently with hydrogen sulfide to give a compound of the formula

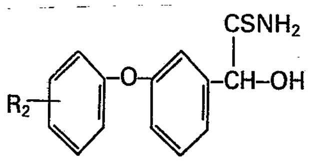

($R_1$ = $-CSNH_2$)

or

c) is reacted with $C_2$-$C_3$alkenyl-MgCl (Br or I), $C_2$-$C_3$alkynyl-MgCl (Br or I), or allenyl-MgCl (Br or I) to give a compound of the formula

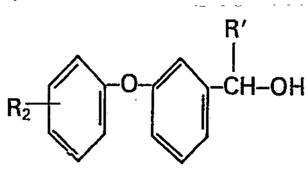

wherein $R_2$ is as defined in claim 1 and R′ is $C_2$-$C_3$alkenyl, $C_2$-$C_3$alkynyl or allenyl.

2. A process according to claim 1 for the preparation of a 3-phenoxybenzyl alcohol of the formula

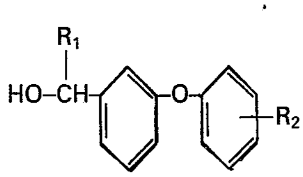

wherein $R_1$ is hydrogen, cyano, allenyl or ethynyl, and $R_2$ is p-vinyl, p-propen-1-yl, p-3-dimethylaminopropen-1-yl, p-ethynyl, p-propyn-1-yl, p-iodoethynyl or p-3-dimethylaminopropyn-1-yl.

3. A process according to claim 2 for the preparation of a 3-phenoxybenzyl alcohol of the formula

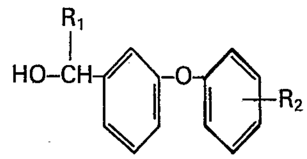

wherein $R_1$ is hydrogen or cyano, $R_2$ is p-vinyl, p-propen-1-yl, p-ethynyl or p-propyn-1-yl.

4. A process according to claim 3 for the preparation of the compound of the formula

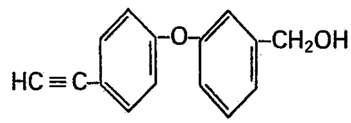

5. A process according to claim 3 for the preparation of the compound of the formula

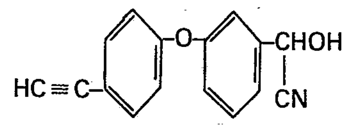

6. A process according to claim 3 for the preparation of the compound of the formula

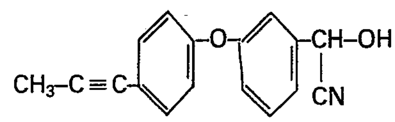

7. A process according to claim 2 for the preparation of the compound of the formula

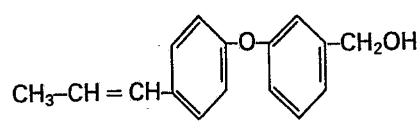

8. A process according to claim 2 for the preparation of the compound of the formula

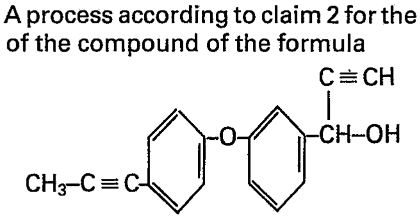

9. A process according to claim 2 for the preparation of the compound of the formula

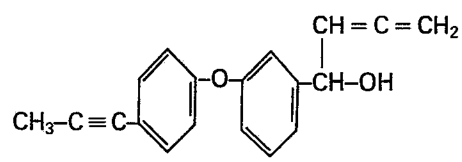

10. A process according to claim 3 for the preparation of the compound of the formula

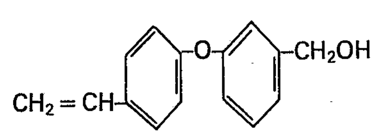

11. A process according to claim 3 for the preparation of the compound of the formula

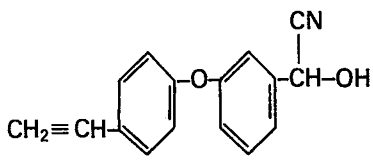

12. A process according to claim 3 for the preparation of the compound of the formula

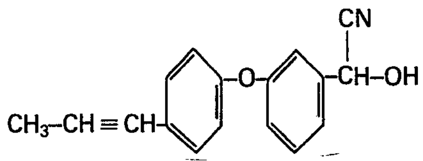

13. A process according to claim 2 for the preparation of the compound of the formula

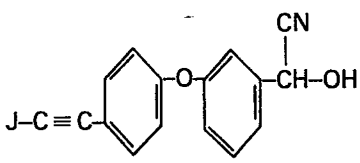

14. A process according to claim 2 for the preparation of the compound of the formula

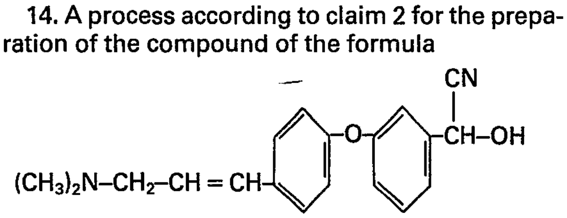

15. A process according to claim 2 for the preparation of the compound of the formula

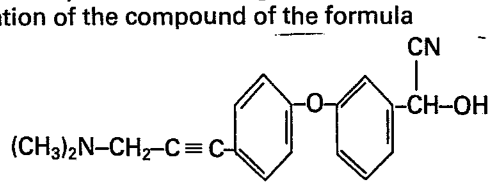

**Revendications pour les Etats contractants: BE, CH, DE, FR, GB, LI, IT, NL**

1. 3-phénoxybenzylalcools de formule

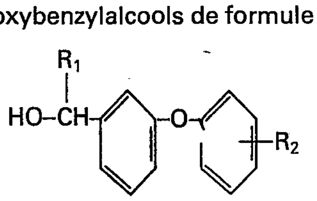

où $R_1$ représente un hydrogène, un cyano, $-CSNH_2$, un allényle, un alcényle en $C_2$ à $C_3$ ou un alcynyle en $C_2$ à $C_3$ et $R_2$ représente un alcényle ou un alcynyle éventuellement substitué par un cyano, un halogène, un alcoyle en $C_1$ à $C_6$, un hydroxyalcoyle en $C_1$ à $C_6$, un halogénalcoyle en $C_1$ à $C_6$ ou un diméthylamino.

2. Composés selon la revendication 1, où $R_1$ représente un hydrogène, un cyano, un allényle ou un éthynyle et $R_2$ représente un p-vinyle, un p-1-propényle, un p-3-diméthylamino-1-propényle, un p-éthynyle, un p-1-propynyle, un p-iodéthynyle ou un p-3-diméthylamino-1-propynyle.

3. Composés selon la revendication 2, où $R_1$ représente un hydrogène ou un cyano et $R_2$ représente un p-vinyle, un p-1-propényle, un p-éthynyle ou un p-1-propynyle.

4. Le composé selon la revendication 3 de formule

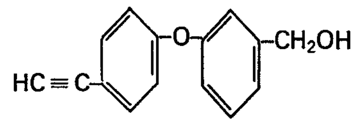

5. Le composé selon la revendication 3 de formule

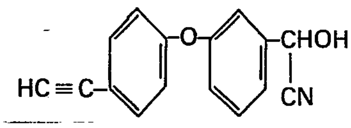

6. Le composé selon la revendication 3 de formule

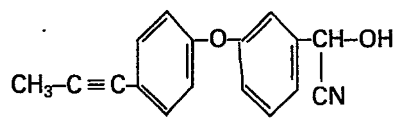

7. Le composé selon la revendication 2 de formule

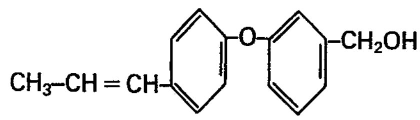

8. Le composé selon la revendication 2 de formule

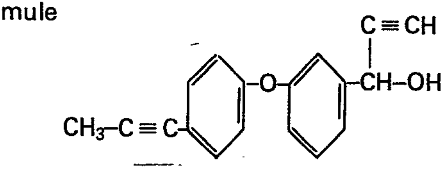

9. Le composé selon la revendication 2 de formule

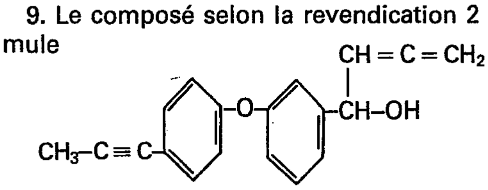

10. Le composé selon la revendication 3 de formule

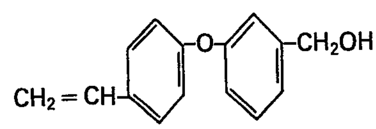

11. Le composé selon la revendication 3 de formule

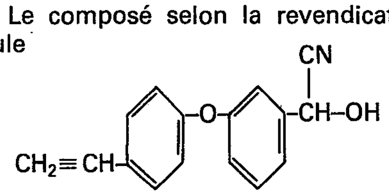

12. Le composé selon la revendication 3 de formule

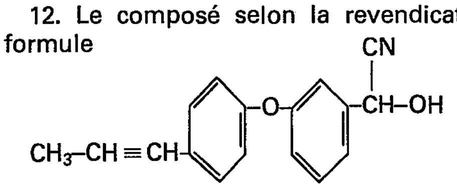

13. Le composé selon la revendication 2 de formule

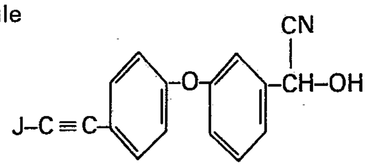

14. Le composé selon la revendication 2 de formule

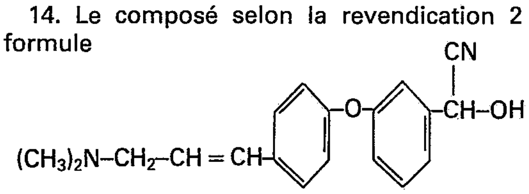

15. Le composé selon la revendication 2 de formule

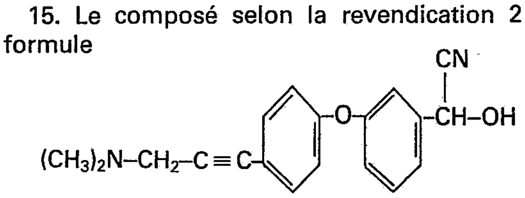

16. Procédé de préparation d'un composé selon la revendication 1, caractérisé en ce qu'on traite de la façon suivante le composé de formule

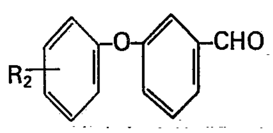

où $R_2$ a la signification donnée dans la revendication 1,

a) On le réduit pour donner un composé de formule

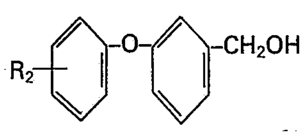 ($R_1$ = hydrogène)

ou

b) On commmence par le faire réagir avec NaCN pour donner un composé de formule

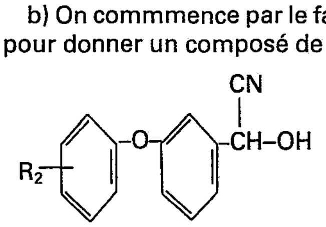 ($R_1$ = cyano)

puis on fait réagir ce composé avec de l'acide sulfhydrique pour donner un composé de formule

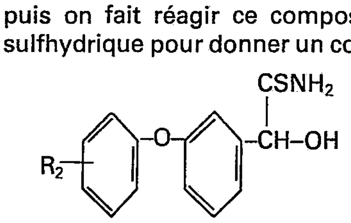 ($R_1$ = $-CSNH_2$)

ou

c) On le fait réagir avec $C_2$-$C_3$-alcényl-MgCl (Br, I), $C_2$-$C_3$-alcynyl-MgCl (Br, I) ou allényl-MgCl (Br, I) pour donner un composé de formule

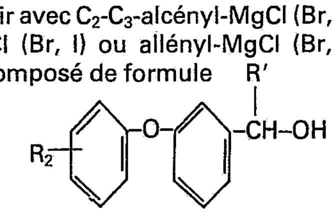

où $R_2$ a la signification donnée dans la revendication 1 et R' représente un alcényle en $C_2$ à $C_3$, un alcynyle en $C_2$ à $C_3$ ou un allényle.

17. 3-phénoxybenzaldéhydes de formule

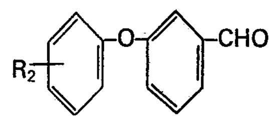

où $R_2$ a la signification donnée dans la revendication 1.

**Revendications pour l'Etat contractant: AT**

1. Procédé de préparation de 3-phénoxybenzylalcools de formule

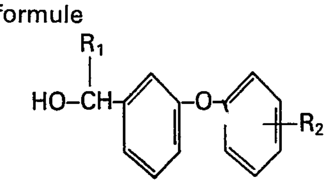

où $R_1$ représente un hydrogène, un cyano, $-CSNH_2$, un allényle, un alcényle en $C_2$ à $C_3$ ou un alcynyle en $C_2$ à $C_3$ et $R_2$ représente un alcényle ou un alcynyle éventuellement substitué par un cyano, un halogène, un alcoyle en $C_1$ à $C_6$, un hydroxyalcoyle en $C_1$ à $C_6$, un halogénalcoyle en $C_1$ à $C_6$ ou un diméthylamino, caractérisé en ce qu'on traite de la façon suivante un composé de formule

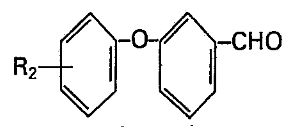

où $R_2$ a la signification donnée ci-dessus,

a) On le réduit pour donner un composé de formule

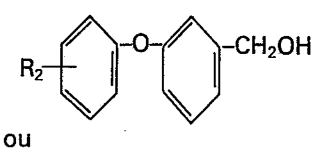 ($R_1$ = hydrogène)

ou

b) On le fait tout d'abord réagir avec NaCN pour donner un composé de formule

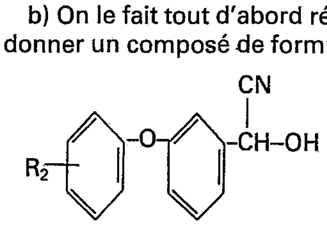 ($R_1$ = cyano)

et on fait réagir ce composé avec de l'acide sulfhydrique pour donner un composé de formule

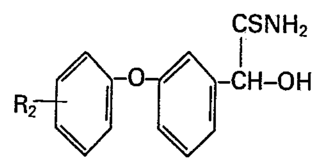 ($R_1$ = $-CSNH_2$)

ou

c) On le fait réagir avec alcényl-MgCl (Br, I), alcynyl-MgCl (Br, I) ou allényl-MgCl (Br, I) pour donner un composé de formule

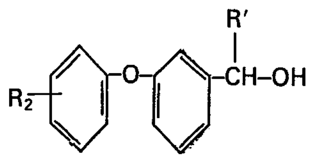

où $R_1$ représente un alcényle, un alcynyle ou un allényle.

2. Procédé de préparation selon la revendication 1 de 3-phénoxybenzyl-alcools de formule

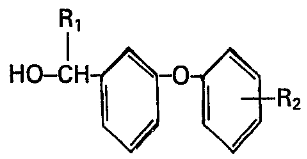

où $R_1$ représente un hydrogène, un cyano, un allényle ou un éthynyle et $R_2$ représente un p-vinyle, un p-1-propényle, un p-3-diméthylamino-1-propényle, un p-éthynyle, un p-1-propynyle, un p-iodéthynyle ou un p-3-diméthylamino-1-propynyle.

3. Procédé de préparation selon la revendication 2 de 3-phénoxybenzylalcools de formule

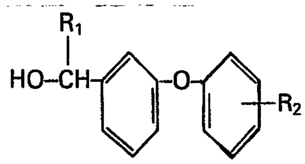

où $R_1$ représente un hydrogène ou un cyano et $R_2$ représente un p-vinyle, un p-1-propényle, un p-éthynyle ou un p-1-propynyle.

4. Procédé de préparation selon la revendication 3 du composé de formule

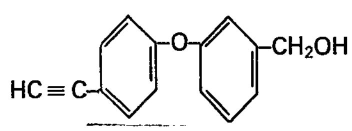

5. Procédé de préparation selon la revendication 3 du composé de formule

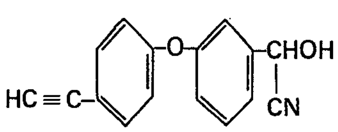

6. Procédé de préparation selon la revendication 3 du composé de formule

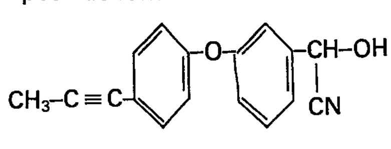

7. Procédé de préparation selon la revendication 2 du composé de formule

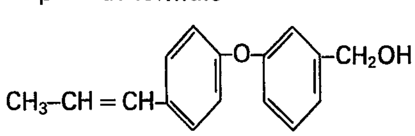

8. Procédé de préparation selon la revendication 2 du composé de formule

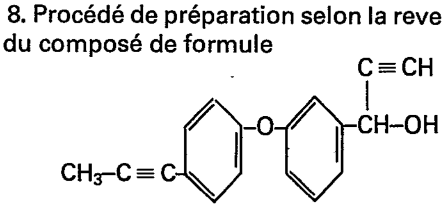

9. Procédé de préparation selon la revendication 2 du composé de formule

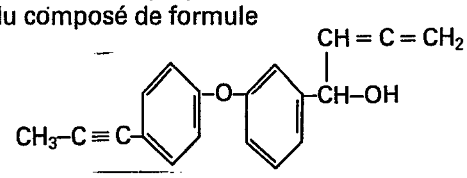

10. Procédé de préparation selon la revendication 3 du composé de formule

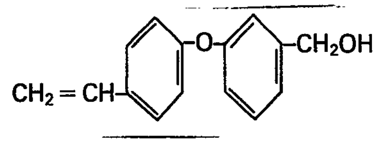

11. Procédé de préparation selon la revendication 3 du composé de formule

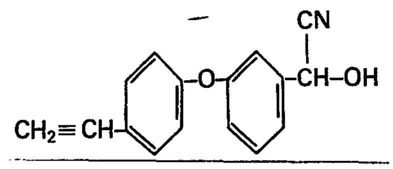

12. Procédé de préparation selon la revendication 3 du composé de formule

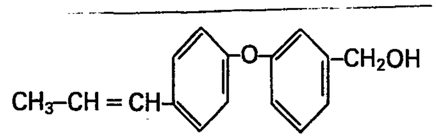

13. Procédé de préparation selon la revendication 2 du composé de formule

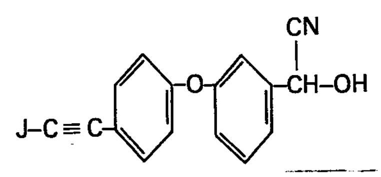

14. Procédé de préparation selon la revendication 2 du composé de formule

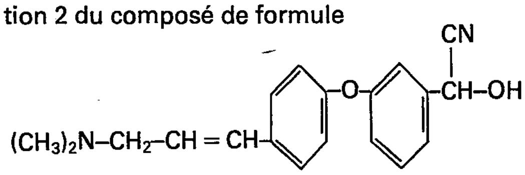

15. Procédé de préparation selon la revendication 2 du composé de formule